# EUROPEAN PATENT APPLICATION

(11) **EP 4 840 952 A1**
(43) Date of publication of application: **18.11.2026**
(21) Application number: 24916245.4
(22) Date of filing: 18.04.2024
(51) Int. Cl.: C12N 9/88, C12N 15/60, C12N 15/70, C12N 1/21, C12P 13/22, C12R 1/19

(54) **AROG MUTANT AND USE THEREOF**

(30) Priority: 11.01.2024 CN 202410042938
(71) Applicant: Hangzhou Ouhe Biotechnology Co., Ltd., Hangzhou, Zhejiang 310018 (CN)
(72) Inventor: WANG, Qiang, Hangzhou, Zhejiang 310018 (CN); GAO, Qilin, Hangzhou, Zhejiang 310018 (CN); ZHENG, Huabao, Hangzhou, Zhejiang 310018 (CN)
(74) Representative: Metida
(86) International application number: PCT/CN2024/088696
(87) International publication number: WO 2025/148187

(57) **Abstract**

The present disclosure relates to the technical field of bioengineering, and particularly to an aroG isoenzyme mutant of 3-deoxy-D-arabino-heptulosonate-7-phosphate (DAHP) synthase and use thereof. Mutants obtained by mutating aroG derived from *Escherichia coli, Klebsiella aerogenes, Proteus mirabilis, Yersinia kristensenii,* or *Cronobacter sakazakii* can increase the yield of a tryptophan gene-edited engineered bacterium, and possess broad application prospects.

## Description

### TECHNICAL FIELD

The present disclosure relates to the technical field of bioengineering, and particularly to an aroG isoenzyme mutant of 3-deoxy-D-arabino-heptulosonate-7-phosphate (DAHP) synthase and use thereof.

### BACKGROUND

In *Escherichia coli*, DAHP synthase, (EC 4.1.2.15) is an enzyme that catalyzes the first step of the aromatic amino acid biosynthesis pathway, and catalyzes condensation of phosphoenolpyruvate and erythrose-4-phosphate to form DAHP and phosphate [Ger YM, Chen SL, Chiang HJ, Shiuan D. A single Ser-180 mutation desensitizes feedback inhibition of the phenylalanine-sensitive 3-deoxy-D-arabino-heptulosonate 7-phosphate (DAHP) synthetase in Escherichia coli. J Biochem. 1994;116(5):986-990.]. DAHP synthase includes three isoenzymes, namely aroG, aroH, and aroF. Among them, the feedback inhibitor of aroG is phenylalanine, the feedback inhibitor of aroH is tryptophan, and the feedback inhibitor of aroF is tyrosine. The three isoenzymes aroG, aroH, and aroF account for 80%, 1%, and 20%, respectively, of the total activity of DAHP synthase [Kikuchi Y, Tsujimoto K, Kurahashi O. Mutational analysis of the feedback sites of phenylalanine-sensitive 3-deoxy-D-arabino-heptulosonate-7-phosphate synthase of Escherichia coli. Appl Environ Microbiol. 1997;63(2):761-762.].

As the industrial scale of amino acid fermentation has gradually expanded, mechanisms for relieving feedback inhibition of DAHP synthase have been extensively studied. Previous studies have proposed two potential mechanisms of feedback inhibition: the amino acid substitution of Asn8 with Lys8 and deletion of the first seven amino acids at the N-terminus of aroF resulted in desensitization of Tyr-sensitive DAHP synthase, indicating that the inhibitor-binding site may be located in the N- or C-terminal domain. Meanwhile, sites near the inhibitor-binding pocket, such as Asp146Asn, Pro150Leu, Ser180Phe, Phe209Ser, and the like, can substantially relieve feedback inhibition of aroG, indicating that phenylalanine induces internal changes in enzyme conformation by binding to the feedback inhibition site [Xu J, Hu C, Shen S, Wang W, Jiang P, Huang W. Requirement of the N-terminus for dimer formation of phenylalanine-sensitive 3-deoxy-D-arabino-heptulosonate synthase AroG of Escherichia coli. J Basic Microbiol. 2004;44(5):400-406.].

### SUMMARY

In order to solve the above technical problems, the present disclosure provides a strategy utilizing rational protein design. By computer simulation calculation and analysis of an inhibitor-binding pocket, amino acid sites around the inhibitor Phe-binding pocket in aroG synthase are screened, thereby obtaining key sites capable of relieving feedback inhibition.

The present disclosure provides an aroG isoenzyme mutant of DAHP synthase, wherein aroG derived from *Escherichia coli*, *Klebsiella aerogenes*, *Proteus mirabilis*, *Yersinia kristensenii*, or *Cronobacter sakazakii* comprises any one of the following mutations: Q151F, Q151F+D146N, Q151F+P150L, Q151F+S180F, Q151F+F209A, Q151F+S211F, or Q151F+S180F+S211F.

Preferably, the amino acid sequence of the aroG isoenzyme mutant comprises any one of the following mutations: Q151F, Q151F+D146N, Q151F+P150L, Q151F+S180F, Q151F+F209A, Q151F+S211F, or Q151F+S180F+S211F, relative to the amino acid sequence set forth in SEQ ID NO: 2, SEQ ID NO: 3, SEQ ID NO: 4, SEQ ID NO: 5, or SEQ ID NO: 6.

The present disclosure provides a gene encoding the aroG isoenzyme mutant, wherein preferably, the nucleotide sequence of the gene comprises any one of the following mutations: Q151F, Q151F+D146N, Q151F+P150L, Q151F+S180F, Q151F+F209A, Q151F+S211F, or Q151F+S180F+S211F, relative to the nucleotide sequence set forth in SEQ ID NO: 1.

The present disclosure further provides a recombinant vector comprising the coding nucleic acid. Preferably, the recombinant vector is a pTrc99a plasmid.

The present disclosure further provides a recombinant engineered bacterium comprising the recombinant vector. Preferably, the recombinant engineered bacterium is *Escherichia coli*. In particular, the recombinant engineered bacterium is used for producing tryptophan.

The present disclosure provides use of the aroG isoenzyme mutant, or a coding gene thereof, in preparing tryptophan.

The present disclosure further provides use of the aroG isoenzyme mutant in the synthesis of tryptophan-supplemented food products, pharmaceuticals, fine chemicals, health products, or cosmetic products.

The aroG isoenzyme mutant of DAHP synthase obtained by the present disclosure can increase the yield of a tryptophan gene-edited engineered bacterium, can be used for preparing tryptophan, and can further be used in the synthesis of food products, pharmaceuticals, fine chemicals, health products, or cosmetic products, and thus possesses broad application prospects.

### BRIEF DESCRIPTION OF THE DRAWINGS

Fig. 1 shows the docking conformation of aroG synthase with the inhibitor Phe in Example 1.
Fig. 2 shows an agarose gel electrophoresis result of the aroG gene in the Polymerase Chain Reaction (PCR) amplification system in Example 2.
Fig. 3 shows an agarose gel electrophoresis image of pTrc99a_aroG constructed in Example 2.
Fig. 4 shows a sodium dodecyl sulfate-polyacrylamide gel electrophoresis (SDS-PAGE) electrophoresis image of aroG wild-type and mutant proteins.
Fig. 5 shows detection results of the feedback inhibition-resistant effect of aroG mutants.
Fig. 6 shows a sequence alignment diagram of aroG from five different bacterial genera.
Fig. 7 shows detection results of the feedback inhibition-resistant effect of aroG multi-site mutants.
Fig. 8 shows a comparison diagram of the feedback inhibition-resistant effect of aroG wild-type and mutants at different inhibitor concentrations.

### DETAILED DESCRIPTION OF THE EMBODIMENTS

The present disclosure is further described below with reference to the drawings and specific examples, so that those skilled in the art can better understand and practice the present disclosure. However, the examples provided are not intended to limit the present disclosure.
1. Relevant nucleotide sequence information in the sequence listing in the examples of the present disclosure:
   The sequence information of SEQ ID NO: 1 is the nucleotide sequence of the DAHP synthase gene aroG of *Escherichia coli* MG1655.

The sequence information of SEQ ID NO: 2 is the amino acid sequence of the DAHP synthase gene aroG of *Escherichia coli* MG1655.

The sequence information of SEQ ID NO: 3 is the amino acid sequence of the DAHP synthase gene aroG of *Klebsiella aerogenes.*

The sequence information of SEQ ID NO: 4 is the amino acid sequence of the DAHP synthase gene aroG of *Proteus mirabilis.*

The sequence information of SEQ ID NO: 5 is the amino acid sequence of the DAHP synthase gene aroG of *Yersinia kristensenii*.

The sequence information of SEQ ID NO: 6 is the amino acid sequence of the DAHP synthase gene aroG of *Cronobacter sakazakii*.

### 2. Materials and methods involved in the examples of the present disclosure:

### (1) Culture media:

Solid medium: peptone 10 g/L, yeast extract 5 g/L, sodium chloride 10 g/L, agar powder 20 g/L.

Seed medium: peptone 10 g/L, yeast extract 5 g/L, sodium chloride 10 g/L.

Fermentation medium: glucose 20 g/L (dispensed), yeast extract 3 g/L, (NH₄)₂SO₄ 4 g/L, KH₂PO₄ 2.5 g/L, sodium chloride 1 g/L, MgSO₄·7H₂O 1 g/L, citric acid 2 g/L, FeSO₄·7H₂O 20 mg/L, MnSO₄·7H₂O 1.2 mg/L, Vitamin H (VH) 0.1 mg/L, Vitamin B₁ (VB₁) 0.5 mg/L, trace element mixture 1 mL/L, phenol red solution 2%, water added to volume, and pH adjusted to 7.0-7.2. The prepared medium was subjected to moist heat sterilization at 121°C and 0.1 MPa for 20 min.

### (2) Shake flask fermentation process

Shake tube activation culture: 100 µL of bacterial culture was aspirated into a shake tube containing 10 mL of Luria-Bertani (LB) liquid medium, and cultured with shaking at 37°C and 220 rpm for about 12 h.

Shake flask seed culture: 600 µL of bacterial culture was inoculated by pipette into a 500 mL Erlenmeyer flask containing 30 mL of seed medium, and cultured at 37°C and 220 rpm for 3-4 h.

Shake flask fermentation culture: 600 µL of seed culture was transferred by pipette into a 500 mL Erlenmeyer flask containing 30 mL of fermentation medium. When the bacteria grew to an OD₆₀₀ value of about 0.8, 0.2 mM isopropyl-β-D-thiogalactopyranoside (IPTG) inducer was added, followed by shaking culture at 37°C and 220 rpm for fermentation for 24 h.

### Example 1: Discovery of feedback inhibition-resistant sites of aroG

First, a three-dimensional crystal structure of aroG synthase (Gene ID: 945605) was obtained from the RCSB Protein Data Bank database (PDB ID: 8e0x), and a three-dimensional structure of the feedback inhibitor phenylalanine (Phe) was obtained from the NCBI PubChem database (CAS: 63-91-2). The structures of the protein receptor aroG and the small molecule ligand Phe were then processed, including removing crystallographic water molecules from the protein structure, removing excess metal ions and other prosthetic groups, coenzymes, and the like from the crystal structure, repairing missing amino acids in peptide chains and missing atoms in amino acids, removing repeated structures in a multimeric protein structure, and performing energy minimization on the protein structure and the small molecule ligand to allow each to reach its most stable state.

Autodock Vina software was then used to perform flexible molecular docking of aroG synthase and Phe. The flexible residues of the aroG protein may be set to sites near the inhibitor Phe-binding pocket, such as Asp146, Pro150, Ser189, Phe209, and the like. The docking box size was set to about 40 Å, and the coordinates of the docking box center were center_x = 34.706, center_y = 414.253, center_z = 39.641. The energy range for docking conformation screening was 0-4, and 20 docking conformations were finally output for analysis and comparison.

Among the 20 output docking conformations, a result having relatively low binding energy and a reasonable docking conformation was selected as a basic conformation for screening feedback inhibition sites, focusing on amino acid residues within 10 Å around the inhibitor Phe-binding pocket. The key to relieving feedback inhibition of aroG lies in reducing binding of the inhibitor Phe. By analyzing the docking conformation of aroG and Phe, the C-terminal carboxyl group and the N-terminal amino group of the inhibitor bind to residues such as Ser180, Asp6, and Asp7 in the active pocket of the aroG protein, thereby transmitting a feedback inhibition signal to the catalytic active center of aroG, causing a change in enzyme conformation, and thus leading to a substantial decrease in enzyme activity. Amino acid sites Q151, A154, and G178, which have relatively small side chains around the inhibitor-binding pocket and are relatively close to the C-terminus, N-terminus, or benzene ring of the inhibitor, were selected and mutated into residues having larger side chains or stronger hydrophobicity, such as Phe or Glu, to block binding of the inhibitor and confer resistance to feedback inhibition. Finally, aroG feedback-resistant mutants A154E, A154F, Q151F, and G178F were obtained by rational design.

### Example 2: Construction of expression vectors pTrc99a-aroG wild type and mutants

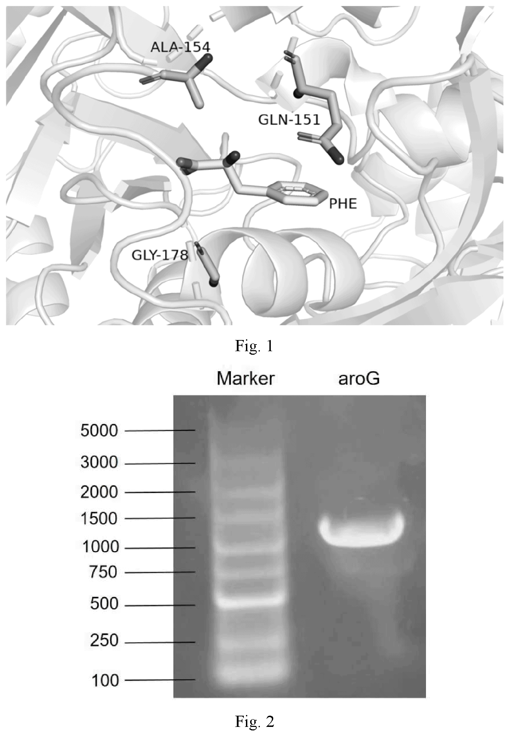

The DAHP synthase gene aroG (Gene ID: 945605) used in the present disclosure was derived from *Escherichia coli* strain MG1655. Genomic DNA of *Escherichia coli* was extracted. According to published genome information and pTrc99a plasmid sequence information, primer pairs aroG-F and aroG-R were designed. Using the extracted genomic DNA of *Escherichia coli* as a template, a standard PCR amplification system and program were used to amplify and obtain the aroG gene. Agarose gel electrophoresis was used to verify the sequence of the amplification product, and Fig. 2 shows the electrophoresis result.

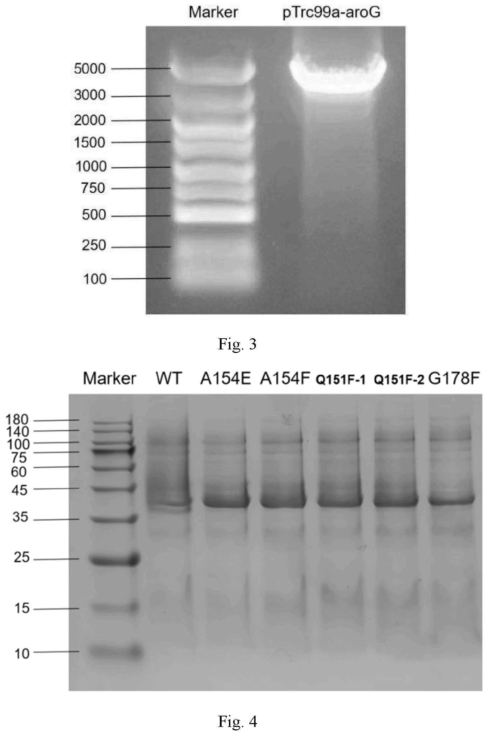

After the sequence size was verified to be correct, Dpn I DNA methylation digestion enzyme was used to digest the PCR system at 37°C for 30 minutes to remove MG1655 template DNA from the system. Next, a nucleic acid purification kit was used to purify the aroG amplification product, removing proteins, salt ions, and small DNA fragments from the system. Primer pairs pTrc99a-F and pTrc99a-R were designed to linearize the pTrc99a plasmid, such that the aroG target gene and the linear vector pTrc99a carried homologous fragments of an appropriate length. The ClonExpress II One Step Cloning Kit from Vazyme was used to ligate the target gene and the vector at 37°C for 30 minutes. After successful ligation, the product was transformed into *Escherichia coli* DH5α. Primer pairs pTrc99a_aroG-F and pTrc99a_aroG-R were designed for sequence verification. The agarose gel electrophoresis image is shown in Fig. 3. After the band size was verified to be correct, the product was sent to a sequencing company for sequencing, and it was verified that the sequence information of the constructed pTrc99a_aroG was correct.

The aroG mutant genes A154E, A154F, Q151F, and G178F used in the present disclosure were obtained by designing site-directed mutagenesis PCR primers (sequence information of the mutation primers is shown in Table 1), using the pTrc99a_aroG wild-type plasmid as a template, and employing a standard PCR amplification system and program to amplify and obtain aroG mutant expression vectors.

All primer sequences included in the construction of aroG wild-type and mutant expression vectors are shown in Table 1.

**Table 1 Relevant Primer Sequences in Example 2**

| Primer name | Primer sequence 5'- 3' |
|---|---|
| aroG-F | TTAAGGAGGCGAAATatgaattatcagaacgacgatttacgcat |
| aroG-R | CTTTTTAAGCTCTAAttacccgcgacgcgc |
| pTrc99a-F | TTAGAGCTTAAAAAGCCCGTCCG |
| pTrc99a-R | ATTTCGCCTCCTTAATCTCTTGTCGT |
| pTrc99a_aroG-F | gtgtcgctcaaggcgcac |
| pTrc99a_aroG-R | tggggtcaggtgggacca |
| A154E-F | ATCTCgaaGACCTGATGAGCTGGGGCGCAATT |
| A154E-R | CATCAGGTCttcGAGATATTGTGGGGTGATCATATCG |
| A154F-F | ATCTCtttGACCTGATGAGCTGGGGCGCAATT |
| A154F-R | CATCAGGTCaaaGAGATATTGTGGGGTGATCATATCG |
| Q151F-F | TCACCCCAtttTATCTCGCTGACCTGATGAGCTG |
| Q151F-R | GAGATAaaaTGGGGTGATCATATCGAGAAACTCAC |
| G178F-F | CGAACTGGCATCAtttCTTTCTTGTCCGGTCGGCT |
| G178F-R | GaaaTGATGCCAGTTCGCGGTGCA |

### Example 3: Detection of feedback inhibition resistance of aroG mutant enzymes

### (1) Culture media:

Solid medium: peptone 10 g/L, yeast extract 5 g/L, sodium chloride 10 g/L, agar powder 20 g/L.

Seed medium: peptone 10 g/L, yeast extract 5 g/L, sodium chloride 10 g/L.

Fermentation medium: glucose 20 g/L (dispensed), yeast extract 3 g/L, (NH₄)₂SO₄ 4 g/L, KH₂PO₄ 2.5 g/L, sodium chloride 1 g/L, MgSO₄·7H₂O 1 g/L, citric acid 2 g/L, FeSO₄·7H₂O 20 mg/L, MnSO₄·7H₂O 1.2 mg/L, VH 0.1 mg/L, VB₁ 0.5 mg/L, trace element mixture 1 mL/L, phenol red solution 2%, water added to volume, and pH adjusted to 7.0-7.2. The prepared medium was subjected to moist heat sterilization at 121°C and 0.1 MPa for 20 min.

### (2) Shake flask fermentation process

Shake tube activation culture: 100 µL of bacterial culture was aspirated into a shake tube containing 10 mL of LB liquid medium, and cultured with shaking at 37°C and 220 rpm for about 12 h.

Shake flask seed culture: 600 µL of bacterial culture was inoculated by pipette into a 500 mL Erlenmeyer flask containing 30 mL of seed medium, and cultured at 37°C and 220 rpm for 3-4 h.

Shake flask fermentation culture: 600 µL of seed culture was transferred by pipette into a 500 mL Erlenmeyer flask containing 30 mL of fermentation medium. When the bacteria grew to an OD₆₀₀ value of about 0.8, 0.2 mM IPTG inducer was added, followed by shaking culture at 37°C and 220 rpm for fermentation for 24 h.

### (3) Verification of induced expression of aroG protein

After strains carrying pTrc99a_aroG wild-type and mutant plasmids were activated, the strains were transferred into primary seed medium at a ratio of 2% for culture. After 3-4 h, the cultures were transferred into fermentation medium at a ratio of 2%. After culture for 2-3 h, the OD₆₀₀ value of the bacterial culture was measured, and when the value was about 0.6-0.8, 0.2 mM IPTG inducer was added. After induced shake flask fermentation for 24 h, samples were taken and subjected to SDS-PAGE electrophoresis to verify the induced expression of aroG protein. The electrophoresis image is shown in Fig. 4. Compared with a chassis strain without plasmid, the expression levels of pTrc99a_aroG wild-type and mutant proteins were relatively high.

### (4) Method for detecting the product tryptophan

Method for detecting the product tryptophan by high-performance liquid chromatography (HPLC): 1 mL of fermentation broth was taken and centrifuged at 12000 rpm for 5 min, and the supernatant was collected. Sample treatment: a 5 g/L L-tryptophan standard was diluted to 0.12, 0.25, 0.5, and 0.75 g/L, respectively, and fermentation broth samples were diluted by appropriate multiples so that the measured values were between the measured values of the standards. Chromatographic separation conditions: the chromatographic column was a C18 column, the mobile phase was a 10% v/v acetonitrile solution, the column temperature was set to 30°C, the flow rate was 0.8 mL/min, the retention time was 10 min, and the detection wavelength was 278 nm.

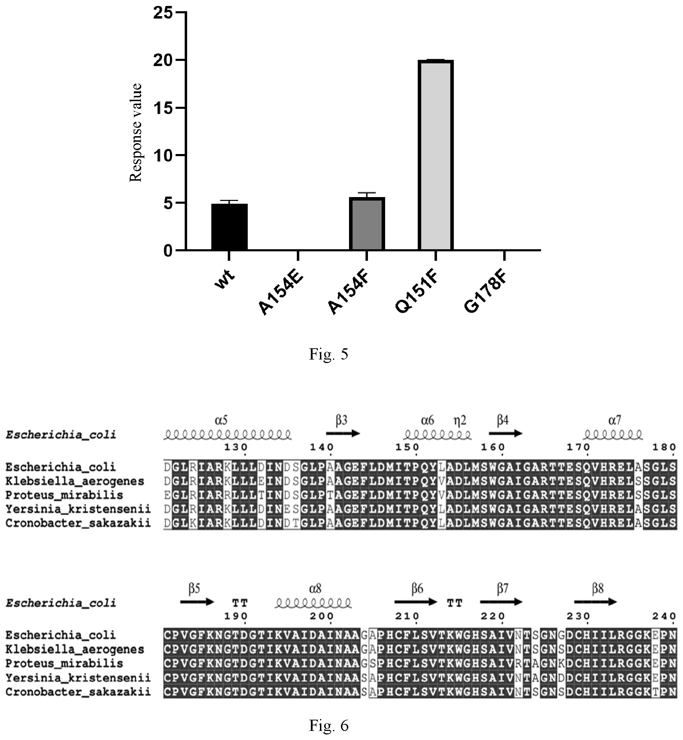

Shake flask fermentation results of aroG wild type and mutants are shown in Fig. 5. Compared with the wild type, the Q151F mutant had a higher yield of the product tryptophan, about 4 times that of the wild type, and had a certain degree of feedback inhibition resistance. The amount of the product tryptophan of A154F was comparable to that of the wild type, indicating that mutation at this position of aroG did not produce a positive effect, the feedback resistance was not enhanced, and the enzyme activity did not significantly change. In the detection results of A154E and G178F mutants, there was no accumulation of the product tryptophan, indicating that directed evolution at these two sites resulted in loss of aroG enzyme activity and failure to generate the product tryptophan.

### Example 4: Screening of feedback inhibition sites by homology alignment of protein sequence structures

The aroG amino acid sequence derived from *Escherichia coli* MG1655 (as set forth in SEQ ID NO: 2) and its protein structure were aligned with the structures of aroG synthase sequences derived from *Klebsiella aerogenes, Proteus mirabilis, Yersinia kristensenii, and Cronobacter sakazakii* (as set forth in SEQ ID NO: 3 to 6, respectively), and the sequence homologies thereof were 92%, 85%, 87%, and 89%, respectively. Observation of the sequence alignment diagram in Fig. 6 shows that, in the α6 region of aroG derived from five different bacterial genera, the Q151 site appears at this site as a conserved amino acid residue. Swiss-PdbViewer software was used to perform virtual mutation of the aroG Q151 site from the five different bacterial genera, changing the site to an F residue. After energy minimization of mutant structures, AutoDock Vina software was used to perform molecular docking of the inhibitor phenylalanine with wild-type and mutant aroG synthases. The docking box size was set to about 40 Å, and the coordinates of the docking box center were center_x = 34.706, center_y = 414.253, center_z = 39.641. The energy range for docking conformation screening was 0-4, and 20 docking conformations were finally output for analysis and comparison. The docking results showed that the affinity between aroG synthase after the Q151F mutation and the inhibitor Phe was lower than the affinity before the mutation. This indicates that the Q151F mutation can produce feedback inhibition resistance in aroG synthases derived from different bacterial genera, and mutations in the α6 region of aroG synthases derived from different bacterial genera are important for improving feedback inhibition resistance and enhancing flux of the Trp metabolic pathway.

### Example 5: Detection of feedback inhibition resistance of aroG multi-site mutant enzymes

First, a strain T29, constructed and preserved in the laboratory and capable of highly producing tryptophan, was used as a chassis. Gene editing was performed by a two-step homologous recombination method to knock out the aroG gene on the chassis strain.

The plasmid pOH5899 used in the following examples was constructed and preserved in the laboratory.

The plasmid pKD46 used in the following examples is a product of miaolingbio (http://www.miaolingbio.com/), product number P0098.

### 5.1 Construction of ΔaroG knockout cassettes aroG-1 and aroG-2

(1) Using plasmid pOH5899 as a template, PCR amplification was performed using primer pair aroG-5899-F/aroG-5899-R, and agarose gel electrophoresis verification was performed to obtain a single target band aroG-cat-SacB.
(2) After the above single target band aroG-cat-SacB was purified and recovered, restriction endonuclease Dpn I was added to digest the methylated plasmid template, and the reaction was performed at 37°C for 30 minutes.
(3) The fragment aroG-cat-SacB after template digestion was purified and verified by sequencing, thereby obtaining a first recombination fragment aroG-1 of the ΔaroG genotype.
(4) Using the laboratory-preserved strain T29 as a template, amplification was performed using primers aroG-up-F and aroG-up-R, agarose gel electrophoresis verification was performed, the template was digested using Dpn I, and purification and recovery were performed to obtain an aroG-up fragment.
(5) Using the laboratory-preserved strain T29 as a template, amplification was performed using primers aroG-down-F and aroG-down-R, agarose gel electrophoresis verification was performed, the template was digested using Dpn I, and purification and recovery were performed to obtain an aroG-down fragment.
(6) Using aroG-up and aroG-down as templates, overlap PCR amplification was performed using primers aroG-up-F and aroG-down-R, agarose gel electrophoresis verification was performed, the template was digested using Dpn I, and purification and recovery were performed to obtain an aroG-2 fragment.

Amplification system: 2×Phanta Max Master Mix (Vazyme) 25 µL, DNA template 20 ng, upstream and downstream primers (10 µM) 1 µL each, and distilled water 20 µL, with a total volume of 50 µL.

Amplification conditions were: pre-denaturation at 95°C for 4 minutes (1 cycle); denaturation at 95°C for 15 seconds, annealing at 56°C for 15 seconds, and extension at 72°C for 1 minute (30 cycles); and extension at 72°C for 5 minutes (1 cycle).

### 5.2 Construction of T29ΔaroG strain

(1) The chassis strain T29 was prepared into chemically competent cells using a conventional preparation method, with reference to the following book: J. Sambrook, D.W. Russell. Molecular Cloning: A Laboratory Manual [M]. Science Press, 2002.
(2) Plasmid pKD46 was transformed into the chassis strain, cultured at 30°C for 10-12 h, and transformants were picked and inoculated into LB medium supplemented with ampicillin and arabinose at final concentrations of 50 mg/L and 2 g/L, respectively.
(3) The inoculated strain was prepared into electrocompetent cells for later use. During competent cell culture, ampicillin and arabinose were added at final concentrations of 50 mg/L and 2 g/L, respectively.
(4) The first recombination fragment aroG-1 was electroporated into the competent cells, plated onto a dual-resistance plate containing chloramphenicol and ampicillin, and cultured overnight at 30°C. Transformants were picked and inoculated into LB medium supplemented with ampicillin, chloramphenicol, and arabinose at final concentrations of 50 mg/L, 25 mg/L, and 2 g/L, respectively, for preparation of electrocompetent cells.
(5) The transformants were prepared into electrocompetent cells for later use. During competent cell culture, ampicillin, chloramphenicol, and arabinose were added at final concentrations of 50 mg/L, 25 mg/L, and 2 g/L, respectively.
(6) The second recombination fragment aroG-2 was electroporated into the competent cells, and 500 µL of incubated bacterial culture was inoculated into a 30 mL Erlenmeyer flask containing sucrose medium, followed by culture at 37°C and 200 rpm for 18-24 h.
(7) After culture, flocculent precipitates may appear in the Erlenmeyer flask. 20 µL was taken from the shake flask and streaked onto a sucrose-containing plate, followed by overnight culture at 37°C. On the next day, single colonies on the plate were picked and replicated onto an LB plate, a chloramphenicol-resistant plate, and an ampicillin-resistant plate. Single colonies that grew on the LB plate but failed to grow on the chloramphenicol-resistant plate and the ampicillin-resistant plate were selected, and colony PCR was performed using primers aroG-VF and aroG-VR. Strains having an amplification length of 1000 bp were positive strains. PCR products were verified by sequencing to obtain the T29ΔaroG strain.

Colony PCR amplification conditions were: pre-denaturation at 95°C for 3 minutes (1 cycle); denaturation at 95°C for 15 seconds, annealing at 56°C for 15 seconds, and extension at 72°C for 1 minute (30 cycles); and extension at 72°C for 5 minutes (1 cycle).

### 5.3 Detection of feedback-resistant performance of aroG multi-site mutant enzymes

(1) After the first round of screening, the feedback-resistant performance of aroG-Q151F was significantly improved compared with the wild type. In order to further improve the feedback-resistant performance and catalytic activity of aroG, other mutation sites having potential positive additive effects were screened in the feedback inhibitor-binding pocket of aroG, including D146N, P150L, S180F, F209A, and S211F. As shown in Fig. 6, these sites are also conserved sites in the aroG amino acid sequences derived from the foregoing five different bacterial genera.
(2) Next, the above mutation sites were superimposed on the basis of the constructed plasmid pTrc99a-aroG-Q151F, and the method for constructing multi-site mutant plasmids was as described in Example 2.
(3) The aroG multi-site mutant plasmids were transformed into the T29ΔaroG strain to obtain T29ΔaroG strains carrying plasmids Q151F+D146N, Q151F+P150L, Q151F+S180F, Q151F+F209A, Q151F+S211F, and Q151F+S180F+S211F.
(4) Finally, differences in feedback inhibition resistance and catalytic activity of different aroG multi-site mutant enzymes were verified by detecting the content of the product tryptophan through shake flask fermentation. The materials and operation process required for the shake flask fermentation experiment and the method for detecting the product tryptophan were as described in Example 3.

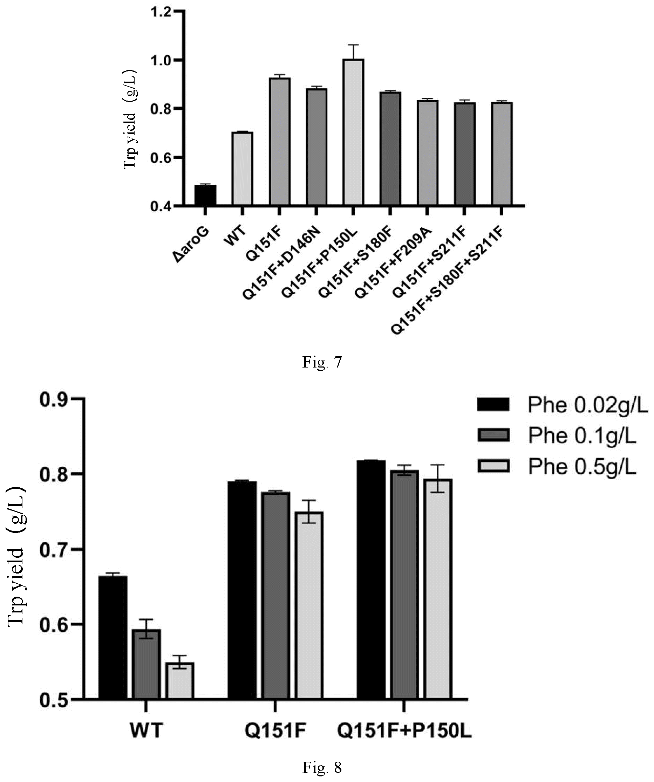

The shake flask fermentation results are shown in Fig. 7. The tryptophan yields accumulated by T29ΔaroG strains carrying aroG wild-type and mutant plasmids after 16 h of shake flask fermentation were significantly higher than that of T29ΔaroG. This reflects that aroG synthase plays an important role in the tryptophan biosynthetic metabolic process, catalyzing condensation of phosphoenolpyruvate and erythrose-4-phosphate to form DAHP and phosphate, promoting metabolic flux toward the aromatic amino acid biosynthesis pathway, and facilitating accumulation of the product tryptophan.

Second, comparison of shake flask fermentation results of aroG wild type and mutants shows that the tryptophan yields accumulated by the single-site mutation Q151F and the multi-site mutations Q151F+D146N, Q151F+P150L, Q151F+S180F, Q151F+F209A, Q151F+S211F, and Q151F+S180F+S211F were all higher than that of wild-type aroG synthase. This indicates that changes at the above sites have a promoting effect on improvement of the feedback inhibition resistance of aroG or enhancement of catalytic activity.

Observation of fermentation results of all aroG multi-site mutant enzymes shows that Q151F+P150L had the highest tryptophan yield relative to the Q151F single-site mutation and other multi-site mutations. This indicates that Q151F+P150L has a better effect than other mutants on enhancing feedback inhibition resistance or catalytic activity of aroG synthase.

In order to further explore the enhancing effect of the Q151F+P150L site mutation on the feedback inhibition resistance of aroG synthase, different concentrations of phenylalanine (an inhibitor of aroG synthase) were additionally added to the initial shake flask medium. The 16 h shake flask fermentation results are shown in Fig. 8.

First, compared with the wild type, strains expressing the Q151F and Q151F+P150L mutant enzymes accumulated higher Trp levels after 16 h shake flask fermentation in media containing different concentrations of inhibitor. This indicates that the aroG mutant enzymes have stronger catalytic activity and can produce more target compound in the same chassis strain, thereby providing higher production efficiency. Second, when the concentration of the inhibitor phenylalanine gradually increased, the decrease in tryptophan yield of Q151F and Q151F+P150L was lower than that of the wild type. This indicates that the inhibitory effect of high concentrations of inhibitor on the aroG mutant enzymes is reduced, and the feedback inhibition resistance of the aroG mutant enzymes is enhanced.

All primer sequences included in the construction of the T29ΔaroG strain and aroG multi-site mutant enzyme expression vectors are shown in Table 2.

**Table 2 Relevant Primer Sequences in Example 5**

| Primer name | Primer sequence 5'-3' |
|---|---|
| aroG-5899-F | AGATCAACGACGGTCTGCGTATAGCCCGTAAATTGCTG CTTGATATTAACTCGAGTGTGACGGAAGATCACTTCGC |
| aroG-5899-R | AGATCAACGACGGTCTGCGTATAGCCCGTAAATTGCTG CTTGATATTAACTCGAGTGTGACGGAAGATCACTTCGC CCGCGCAGAATGATATGGCAATCGCCGTTACCGCTGGT ATTCACAATCGCTCCGGTAGTCAATATCGGCAGAGCTC |
| aroG-up-F | GGATATCTTTACGCCGAAGTGCCC |
| aroG-up-R | TCACAATCGCGTTAATATCAAGCAGCAATTTACGGG |
| aroG-down-F | TGATATTAACGCGATTGTGAATACCAGCGGTAAC |
| aroG-down-R | GCACGAAGGCTTTCCAGCG |
| aroG-VF | GCAATATCGGGTGCTGACCG |
| aroG-VR | GCGTTGATCACGTCTTCAGTCAGA |
| D146N-F | GTTTCTCaatATGATCACCCCAtttTATCTCGCTGACCTGAT GAGCTG |
| D146N-R | GTGATCATattGAGAAACTCACCTGCCGCTG |
| P150L-F | CACCctgtttTATCTCGCTGACCTGATGAGCTG |
| P150L-R | GCGAGATAaaacagGGTGATCATATCGAGAAACTCACCT |
| S180F-F | AGGGCTTtttTGTCCGGTCGGCTTCAAAAATG |
| S180F-R | CCGGACAaaaAAGCCCTGATGCCAGTTCG |
| F209A-F | GCCGCACTGCgcaCTGTCCGTAACGAAATGG |
| F209A-R | GGACAGtgcGCAGTGCGGCGCACCGGC |
| S211F-F | GCTTCCTGtttGTAACGAAATGGGGGCATTCG |
| S211F-R | CGTTACaaaCAGGAAGCAGTGCGGCGC |

Obviously, the above examples are merely examples provided for clear description and are not intended to limit the embodiments. For a person of ordinary skill in the art, other different forms of changes or modifications may be made on the basis of the above description. It is unnecessary and impossible to exhaustively list all embodiments herein. Obvious changes or modifications derived therefrom still fall within the scope of protection of the present disclosure.

## Claims

1. An aroG isoenzyme mutant of 3-deoxy-D-arabino-heptulosonate-7-phosphate (DAHP) synthase, wherein aroG derived from *Escherichia coli*, *Klebsiella aerogenes*, *Proteus mirabilis*, *Yersinia kristensenii*, or *Cronobacter sakazakii* comprises any one of the following mutations: Q151F, Q151F+D146N, Q151F+P150L, Q151F+S180F, Q151F+F209A, Q151F+S211F, or Q151F+S180F+S211F.

2. The aroG isoenzyme mutant of claim 1, wherein the amino acid sequence of the aroG isoenzyme mutant comprises any one of the following mutations: Q151F, Q151F+D146N, Q151F+P150L, Q151F+S180F, Q151F+F209A, Q151F+S211F, or Q151F+S180F+S211F, relative to the amino acid sequence set forth in SEQ ID NO: 2, SEQ ID NO: 3, SEQ ID NO: 4, SEQ ID NO: 5, or SEQ ID NO: 6.

3. A coding nucleic acid encoding the aroG isoenzyme mutant of claim 1 or 2, wherein the nucleotide sequence of the coding nucleic acid comprises any one of the following mutations: Q151F, Q151F+D146N, Q151F+P150L, Q151F+S180F, Q151F+F209A, Q151F+S211F, or Q151F+S180F+S211F, relative to the nucleotide sequence set forth in SEQ ID NO: 1.

4. A recombinant vector comprising the coding nucleic acid of claim 3.

5. The recombinant vector of claim 5, wherein the recombinant vector is a pTrc99a plasmid.

6. A recombinant engineered bacterium comprising the recombinant vector of claim 4.

7. The recombinant engineered bacterium of claim 6, wherein the recombinant engineered bacterium is *Escherichia coli*.

8. The recombinant engineered bacterium of claim 6, wherein the recombinant engineered bacterium is used for producing tryptophan.

9. Use of the aroG isoenzyme mutant of claim 1, or a coding nucleic acid encoding the same, in preparing tryptophan.

10. Use of the aroG isoenzyme mutant of claim 1 in the synthesis of a tryptophan-supplemented food product, pharmaceutical, fine chemical, health product, or cosmetic product.
